# EUROPÄISCHE PATENTANMELDUNG

(11) **EP 2 600 144 A1**
(43) Veröffentlichungstag der Anmeldung: **05.06.2013**
(21) Anmeldenummer: 12194804.6
(22) Anmeldetag: 29.11.2012
(51) Int. Cl.: G01N 25/04, B01L 3/04, G01N 1/12, G01N 1/28, G01N 33/20

(54) **Probentiegel und Verfahren zur Thermoanalyse einer Gießschmelzenprobe**

(30) Priorität: 01.12.2011 DE 102011055950
(71) Anmelder: Fritz Winter Eisengiesserei GmbH & Co. KG, 35260 Stadtallendorf (DE)
(72) Erfinder: Billasch, Jörg, 35279 Neustadt-Mengsberg (DE)
(74) Vertreter: Cohausz & Florack

(57) **Zusammenfassung**

Die vorliegende Erfindung betrifft einen Probentiegel zur Thermoanalyse einer Gießschmelzenprobe, mit einem Probentiegelgefäß (2), in das eine Ausnehmung (3) eingeformt ist, in die die Gießschmelzenprobe einfüllbar ist, wobei in der Ausnehmung (3) ein Impfmittel zum Impfen der Gießschmelzenprobe vorgesehen ist und ein Verfahren zur Thermoanalyse einer Gießschmelzenprobe. Der erfindungsgemäße Probentiegel zur Thermoanalyse einer Gießschmelzenprobe lässt sich kostengünstig herstellen und gewährleistet optimal reproduzierbare Untersuchungsergebnisse. Das erfindungsgemäße Verfahren liefert auf praxisgerechte Weise sicher reproduzierbare Messergebnisse bei verminderten Kosten. Dies wird erfindungsgemäß dadurch erreicht, dass das Impfmittel (8) mittels eines Klebstoffs (7) in der Ausnehmung (3) fixiert ist, der sich neutral gegenüber dem Impfmittel (8) und der Schmelze verhält.

## Beschreibung

Die Erfindung betrifft einen Probentiegel und ein Verfahren zur Thermoanalyse einer Gießschmelzenprobe. Solche Probentiegel und Verfahren werden zur Ermittlung von Zeit-Temperatur-Kurven und der daraus resultierenden Morphologie des Graphits der jeweils untersuchten Legierung eingesetzt. Zu diesem Zweck weisen die Probentiegel eine Ausnehmung auf, die von Feuerfestwerkstoff umgeben ist und in die die Gießschmelzenprobe im schmelzflüssigen Zustand eingefüllt wird. In der Ausnehmung des Probentiegels ist dabei ein Impfmittel zum Impfen der Gießschmelzenprobe vorgesehen. Dieses Impfmittel wird bei der Untersuchung von bestimmten Schmelzen benötigt, um das Abkühl- und Erstarrungsverhalten der untersuchten Legierung direkt zu beeinflussen.

Typische Beispiele für Gießschmelzen, für deren Thermoanalyse Probentiegel der hier in Rede stehenden Art verwendet werden, sind an erster Stelle Eisengussschmelzen, Stahlschmelzen, jedoch auch Leicht- und Schwermetallschmelzen und desgleichen. Durch Zugabe von Impflegierungen auf Siliziumbasis, mit Beimengungen von Antimon, Wismut, Calcium, Aluminium und anderen Elementen, kann der Verlauf der Abkühlkurve der Gussschmelze beeinflusst werden (s. Gießerei Lexikon, Ausgabe 2008. 19. Auflage, Seiten 605 - 609, Fachverlag Schiele & Schön, Berlin).

Aus der DE-OS 1 798 004 ist es allgemein bekannt, wie eine Thermoanalyse in einem Probentiegel untersucht werden kann und wie ein Impfmittel der zu untersuchenden Schmelzenprobe zugesetzt werden kann, um deren Erstarrungsverhalten zu beeinflussen. Das Impfmittel wird demnach in einer ersten Variante entweder in zerkleinerter Form vor oder unmittelbar nach dem Abstich der schmelzflüssigen Gusseisenprobe dem Probentiegel zugegeben. Alternativ kann der Probentiegel an einer der Flächen, die seine zur Aufnahme der Probe bestimmte Ausnehmung umgibt, mit dem Impfmittel beschichtet sein.

Praktische Erfahrungen haben gezeigt, dass eine in dieser Weise erfolgende Zugabe des Impfmittels verschiedene Probleme nach sich zieht, die zu Messungenauigkeiten führen können. So kann es einerseits bei einer Impfung der Schmelze während und unmittelbar nach dem Einfüllen in den Probentiegel zu einer ungleichmäßigen Verteilung des Impfmittels kommen. Andererseits kann es bei einer Beschichtung einer die Ausnehmung begrenzenden Wandfläche mit dem Impfmittel zu Ausgasungen in größerem Umfang in Folge des schlagartig in großen Mengen frei werdenden, zum Binden des Impfstoffs benötigten Binders kommen.

Um diese Probleme zu lösen ist in der DE 26 46 539 ein für die Thermoanalyse von Gusseisen oder Stahlschmelzen vorgesehener Tiegel vorgeschlagen worden, der aus einem Formsand hergestellt ist und bei dem auf einem beschränkten Abschnitt der Wandung, die die zur Aufnahme der zu untersuchenden Probe bestimmte Ausnehmung umgibt, ein Gemisch angebracht ist, das aus einem Impfstoff, einem Wasserstoff erzeugenden Material und einem feuerfesten Material besteht. Wenn die in den Probentiegel gefüllte Schmelzenprobe in Kontakt mit dem Gemisch kommt, wird das Impfmittel freigesetzt und kann so seine Wirkung entfalten. Durch seine auf einen bestimmten Abschnitt der Ausnehmung des Tiegels beschränkte Anordnung wird zudem erreicht, dass das Impfmittel verlangsamt freigesetzt wird. Hierdurch wird beispielsweise die Bildung von Gasen vermieden, durch die das Analyseergebnis verschlechtert werden würde.

Das Auftragen des Gemisches auf den betreffenden Wandabschnitt erfolgt bei dem bekannten Probentiegel bereits bei der Herstellung des Probentiegels. Nach dem Auftrag ist das Gemisch unlösbarer Bestandteil des Probentiegels. Entsprechend ausgebildete Probentiegel werden auch heute noch angeboten (Broschüre "Thermal Analysis of Cast Iron" der Heraeus Electro-Nite International N.V., Houthalen, Belgien, www.electro-nite.be).

Neben dem voranstehend erläuterten Stand der Technik ist es aus der DE-PS 27 39 159 bekannt, in einen Probentiegel für die Thermoanalyse ein Impfmittel als Ablagerung anzuordnen. Das Impfmittel kann dabei als Perlen oder schichtweise auf der Tiegelbodenfläche angeordnet werden. Dabei geht dieser Vorschlag davon aus, dass das Impfmittel erst bei der Probennahme der Probe zugesetzt wird und dass das mit einem geeigneten Bindemittel gebundene Impfmittel selbsttätig am Boden der Ausnehmung des Probentiegels haftet.

Vor dem Hintergrund des voranstehend erläuterten Standes der Technik bestand die Aufgabe der Erfindung darin, einen Probentiegel für die Thermoanalyse von Gießschmelzenproben zu schaffen, der kostengünstig herstellbar ist und mit dem sich optimal reproduzierbare Untersuchungsergebnisse gewährleisten lassen. Ebenso sollte ein Verfahren zur Thermoanalyse angegeben werden, das auf praxisgerechte Weise sicher reproduzierbare Messergebnisse bei verminderten Kosten liefert.

In Bezug auf den Probentiegel ist diese Aufgabe erfindungsgemäß dadurch gelöst worden, dass ein solcher Probentiegel die in Anspruch 1 angegebenen Merkmale aufweist.

In Bezug auf das Verfahren löst die Erfindung die oben genannte Aufgabe dadurch, dass bei der Thermoanalyse einer Schmelzenprobe die in Anspruch 11 angegebenen Arbeitsschritte absolviert werden.

Ein erfindungsgemäßer Probentiegel zur Thermoanalyse einer Gießschmelzenprobe umfasst demnach in Übereinstimmung mit dem eingangs erläuterten Stand der Technik ein Probentiegelgefäß, in das eine Ausnehmung eingeformt ist, in die die Gießschmelzenprobe einfüllbar ist, wobei in der Ausnehmung ein Impfmittel zum Impfen der Gießschmelzenprobe vorgesehen ist.

Erfindungsgemäß ist nun das Impfmittel mittels eines Klebstoffs in der Ausnehmung fixiert, der sich neutral gegenüber dem Impfmittel und der Schmelze verhält.

Das in der erfindungsgemäßen Weise im Probentiegel eingeklebte Impfmittel ist dabei insbesondere als fester, separat vorgefertigter Körper oder aber auch in schüttfähiger Form, beispielsweise als Pulver oder Granulat, bereitgestellt.

Für einen erfindungsgemäßen Tiegel wird ein Impfmittel bereit gestellt, das entweder in schüttfähiger Form, also beispielsweise als Pulver oder Granulat, oder als fester Körper, also beispielsweise als Block oder Scheibe, bereitgestellt wird, der das Ergebnis eines gesonderten Arbeitsgangs ist und beispielsweise nach Art einer Tablette oder desgleichen ausgebildet ist.

Ebenso wird das Probentiegelgefäß mit seiner zur Aufnahme der Gießschmelzenprobe bestimmten Ausnehmung getrennt hergestellt und ist dementsprechend unabhängig von dem Impfmittel handhabbar.

Die getrennte Bereitstellung von Probentiegelgefäß und Impfmittel hat den wesentlichen Vorteil, dass es auf einfache Art und Weise mit minimiertem Aufwand möglich ist, das Impfmittel an einer für die jeweils gewünschte Wirkung optimalen Stelle in der Ausnehmung des Probentiegels zu positionieren. Die erfindungsgemäße Aufteilung erlaubt es auf diese Weise, das Impfmittel bereits im Herstellungsprozess des Tiegels an beliebiger Stelle im Tiegel durch den Klebstoff mit Hilfe einer Montagemaschine zu befestigen. Dabei ist es unerheblich, ob erst das Impfmittel in den Tiegel gegeben und dann mit dem Klebstoff an der geforderten Stelle fixiert wird oder zunächst der Klebstoff eingebracht wird, auf den dann Impfmittel gegeben wird, um es zu fixieren.

Das Impfmittel wird somit erfindungsgemäß an geeigneter Stelle an eine die Aufnahme des Probentiegels umgebende Fläche geklebt. Als Klebstoff kommt dabei ein "Klebstoff" zum Einsatz, der sich gegenüber dem Impfmittel und der zu untersuchenden Gießschmelzenprobe neutral verhält, der also weder die chemische Zusammensetzung der Gießschmelzenprobe wirksam verändert, noch die Wirkung des Impfmittels oder das Erstarrungsverhalten der Gießschmelze beeinflusst.

Der wesentliche Vorteil der erfindungsgemäßen Fixierung des Impfmittels in der Ausnehmung eines Probentiegelgefäßes besteht darin, dass das Impfmittel bereits beim Hersteller im Probentiegel positioniert und die dort positionierte Menge an Impfmittel ebenso exakt eingestellt werden kann.

Das Impfmittel wird bei Kontakt mit der zu untersuchenden Schmelzenprobe schnell aufgelöst und von der Schmelze aufgenommen. Ein erforderlichenfalls zum Binden des Impfmittels benötigter Binder wird durch den Kontakt mit der Schmelze ebenso vollständig aufgelöst oder verbrannt wie der Klebstoff, der erfindungsgemäß zum Fixieren des Impfmittels in der Aufnahme des Probentiegels verwendet wird.

Am Ende der Thermoanalyse ist die in den Probentiegel gefüllte Gießschmelzenprobe zu einem festen Block erstarrt. Aufgrund der durch die Erfindung ermöglichten exakten Bestimmung der Lage und Menge des Impfmittels in der Ausnehmung des Probentiegelgefäßes ist auf denkbar einfache Weise eine sichere Reproduzierbarkeit der Messergebnisse gewährleistet.

Der voranstehend skizzierten, durch die erfindungsgemäße Gestaltung eines Probentiegels ermöglichten Arbeitsweise entsprechend umfasst das erfindungsgemäße Verfahren zur Thermoanalyse einer Gießschmelzenprobe folgende Arbeitsschritte:
a) Bereitstellen eines separat vorgefertigten Impfmittels;
b) Bereitstellen eines separat vorgefertigten Probentiegelgefäßes, das eine Ausnehmung zur Aufnahme der Gießschmelzenprobe aufweist;
c) Fixieren des Impfmittels an einer bestimmten Position innerhalb der Ausnehmung des Probentiegelgefäßes mittels eines Klebstoffs, der sich in Bezug auf das Impfmittel und die Gießprobenschmelze neutral verhält.

Der erfindungsgemäß verwendete Klebstoff sollte so beschaffen sein, dass er das Impfmittel auch dann noch ausreichend fest in der Ausnehmung des Probentiegels hält, wenn der Probentiegel gewissen Erschütterungen ausgesetzt ist, wie sie beim Transport vom Hersteller zum Nutzer oder im Laborbetrieb auftreten. Optimalerweise ist der erfindungsgemäß zum Befestigen des Impfmittels vorgesehene Klebstoff dabei so ausgebildet, dass er durch den Kontakt mit der zu untersuchenden Schmelzenprobe weitestgehend rückstandslos verbrennt.

Überraschender Weise hat sich herausgestellt, dass sich als Klebstoff besonders Stoffe eignen, die zur Gruppe der Saccharide gehören. Hierzu zählen verschiedene Zucker, insbesondere Glukose, Fruchtzucker, Rohr- oder Rübenzucker, jedoch auch Stärke, die nach Art von Kleister oder desgleichen ebenfalls als Kleber eingesetzt werden. Diese erfindungsgemäß zum Fixieren verwendeten Stoffe lassen sich beispielsweise in Wasser lösen und als fließfähige Masse tropfenweise an die jeweils vorgesehene Stelle der Ausnehmung des Probentiegelgefäßes auftragen.

Praktische Versuche haben hier gezeigt, dass sich mit aus handelsüblichem Rohr- oder Rübenzucker hergestelltem Zuckerwasser besonders gute Ergebnisse erzielen lassen. Denkbar ist es beispielsweise bei Verwendung von solchem Zucker jedoch auch, diesen aufzuschmelzen und tropfenweise auf die gewünschte Befestigungsstelle aufzutragen.

Unabhängig davon, wie der Auftrag des Klebstoffs erfolgt, kann das jeweilige Impfmittel einfach auf die an der jeweiligen Stelle der Aufnahme des Probentiegels bereits aufgetragene Zuckermasse aufgebracht oder das an der betreffenden Stelle der Aufnahme des Probentiegels bereits vorhandene Impfmittel mit Zuckermasse übergossen werden. So ist es denkbar, eine auf einen bestimmten Flächenabschnitt aufgetragene Zuckerschicht mit einem schüttfähigen Impfmittel, beispielsweise einem Impfmittelpulver oder -granulat zu bestreuen oder einen in Blockform vorliegenden Impfmittelkörper auf die Zuckerschicht zu setzen. Genauso ist es möglich, zunächst ein schüttfähiges Impfmittel oder einen festen Impfmittelkörper auf den Abschnitt zu setzen und das Impfmittel bzw. den Impfmittelkörper anschließend mit der Zuckermasse zu übergießen.

Unabhängig davon, welche Gestalt das Impfmittel hat, hält die Zuckermasse nach dem Trocknen oder Erstarren das Impfmittel fest an seiner jeweiligen Position in der Ausnehmung.

Die Wahl der jeweiligen Befestigungsposition kann beispielsweise unter Berücksichtigung der jeweiligen Strömungsverhältnisse, die sich beim Einfüllen der Schmelzenprobe in die Ausnehmung des Probentiegels ergeben, so gewählt werden, dass das Impfmittel möglichst schnell in die Schmelze übergeht. Hierzu kann es zweckmäßig sein, das Impfmittel mittels des Klebstoffs an der Bodenfläche der Ausnehmung zu fixieren.

Da bei einem erfindungsgemäßen Probentiegel ein separat vorgefertigtes Impfmittel vorgesehen ist, ist es ebenso einfach möglich, dem Impfmittel eine Form zu geben, durch die die jeweils gewünschte Art der Freisetzung des Impfmittels begünstigt wird. So kann ein fester Impfmittelkörper scheiben-, quader- oder würfelförmig sein oder jede andere freie, durch ein bestimmtes Körpervolumen gekennzeichnete Form haben. Ebenso kann das Impfmittel schüttfähig in Form eines Pulvers, Granulats oder in Form von kleinen Perlen vorliegen.

Abhängig vom zur Durchführung der Thermoanalyse verwendeten Messgerät kann der Probentiegel in an sich bekannter Weise ein an eine Messeinrichtung anschließbares Thermoelement zum Erfassen der Temperatur der Gießschmelzenprobe umfassen. Dieses Thermoelement lässt sich in an sich ebenfalls bekannter Weise so anordnen, dass es mit der Schmelzenprobe in Kontakt kommt und den Temperaturverlauf bei der Abkühlung an das jeweilige Messgerät liefert.

Das Probentiegelgefäß eines erfindungsgemäßen Probentiegels sollte wie der Stand der Technik einerseits in der Lage sein, die beim Einfüllen der Schmelzenprobe in seine Ausnehmung auftretenden thermischen Belastungen sicher zu ertragen. Andererseits sollte das Probentiegelgefäß eine möglichst hohe Verschleißfestigkeit besitzen. Die thermische Belastbarkeit kann dadurch gewährleistet werden, dass die Ausnehmung des Probentiegels von Feuerfestwerkstoff umgeben ist. Eine ausreichende Materialfestigkeit vorausgesetzt, kann dabei das Probentiegelgefäß insgesamt aus Feuerfestwerkstoff bestehen. Es ist jedoch auch denkbar, das die Ausnehmung umgebende Feuerfestmaterial im Sinne einer Auskleidung in einem beispielsweise aus Stahl bestehenden Gerüst zu halten, um eine höhere mechanische Belastbarkeit zu gewährleisten.

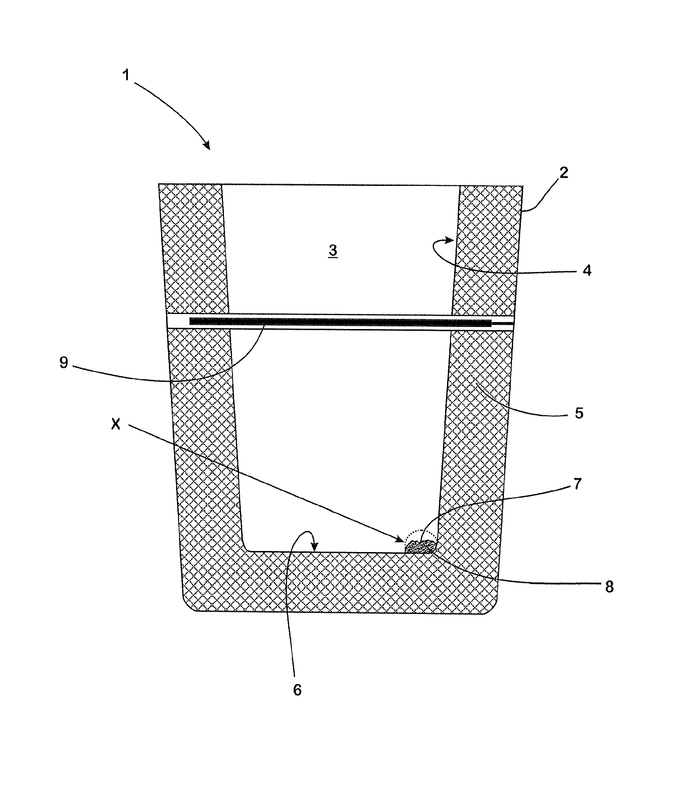

Nachfolgend wird die Erfindung anhand von Ausführungsbeispielen näher erläutert. Die einzige Figur zeigt einen Probentiegel für die Analyse einer Eisengussschmelze in einem Längsschnitt.

Der Probentiegel 1 umfasst ein aus Feuerfestwerkstoff bestehendes Probentiegelgefäß 2. In das Probentiegelgefäß 2 ist ausgehend von dessen oberer Stirnseite eine Ausnehmung 3 eingeformt, so dass das Probentiegelgefäß 2 eine insgesamt topfförmige Gestalt hat. Die Ausnehmung 3 ist dabei umfangsseitig von den Innenflächen 4 der sich in Richtung der Einfüllöffnung der Ausnehmung 3 trichterförmig erweiternden Umfangswand 5 des Probentiegelgefäßes 2 umgrenzt, während sie bodenseitig durch eine ebene Bodenfläche 6 abgeschlossen ist.

Auf der Bodenfläche 6 ist im Bereich des Übergangs zu der angrenzenden Innenfläche 4 mittels eines dünn aufgetragenen Klebstoffs 7 ein Impfmittel 8 an einer bestimmten Position X fixiert. Das Impfmittel 8 kann dabei als Pulver oder Granulat auf einen an der Position X vorgesehenen Flächenabschnitt gestreut und anschließend mit dem Klebstoff 7 übergossen worden sein. Genauso kann das Impfmittel 8 als fester, beispielsweise als Zylinderscheibe ausgebildeter Körper auf eine auf den betreffenden Abschnitt aufgestrichene Klebstoffschicht aufgesetzt sein oder ein pulverförmiger Klebstoff auf eine solche Klebstoffschicht aufgestreut worden sein.

Bei dem Klebstoff 7 handelt es sich hier um handelsüblichen Rohr- oder Rübenzucker, der abhängig von der jeweiligen Art und Weise der Fixierung des Impfmittels 8 entweder zuerst an der Position X aufgetragen und anschließend mit dem Impfmittel 8 belegt oder über das bereits an der Position X angeordnete Impfmittel 8 gegossen worden ist. In beiden Fällen ist nach dem Trocknen das Impfmittel 8 mittels des Klebstoffs 7 fest an der Position X gehalten.

Mit Abstand zur Bodenfläche 6 ist in das Probentiegelgefäß 2 ein Thermoelement 9 eingesetzt, das achsparallel zur Bodenfläche 6 ausgerichtet ist und sich über die gesamte Breite der Ausnehmung 3 zwischen den Umfangswänden 5 des Probentiegelgefäßes 2 erstreckt. Das Thermoelement 9 sitzt in an sich bekannter Weise in einem Glasröhrchen, durch das es gegen die Hitze der in die Ausnehmung 3 zu füllenden Gießschmelzenprobe geschützt ist. Über ein ebenfalls in an sich bekannter Weise seitlich aus der Umfangswand 5 geführtes Anschlusskabel kann das Thermoelement 9 an ein hier nicht gezeigtes Messgerät angeschlossen werden.

Das Probentiegelgefäß 2 besteht üblicherweise aus Formsand, der durch einen geeigneten Binder gebunden ist. Insbesondere eignen sich hierfür Binder, die unempfindlich gegen Feuchtigkeit sind und so eine unproblematische Lagerung des Probentiegels 1 gewährleisten.

Als Impfmittel wird typischerweise Ferrosilizium verwendet.

Für die Thermoanalyse wird die jeweils zu untersuchende Probe einer Eisengussschmelze in die Ausnehmung 3 gefüllt. Die Eisengussschmelze trifft dabei auf das Impfmittel 8, das sich daraufhin schnell auflöst, so dass das in ihm enthaltene Impfmittel in Folge der durch das Einfüllen verursachten Bewegungen der Eisengussschmelze gleichmäßig in der zu untersuchenden Probe verteilt. Der Temperaturverlauf der Erstarrung wird vom Thermoelement 9 erfasst und an die nicht gezeigte Messeinrichtung geliefert.

### BEZUGSZEICHEN

- 1: Probentiegel
- 2: Probentiegelgefäß
- 3: Ausnehmung des Probentiegelgefäßes 2
- 4: Innenfläche der Umfangswand 5
- 5: Umfangswand des Probentiegelgefäßes 2
- 6: Bodenfläche des Probentiegelgefäßes 2
- 7: Klebstoff
- 8: Impfmittel
- 9: Thermoelement

- X: Position des Impfmittelkörpers 8 in der Ausnehmung 3 des Probentiegelgefäßes 2

## Patentansprüche

1. Probentiegel zur Thermoanalyse einer Gießschmelzenprobe, mit einem Probentiegelgefäß (2), in das eine Ausnehmung (3) eingeformt ist, in die die Gießschmelzenprobe einfüllbar ist, wobei in der Ausnehmung (3) ein Impfmittel zum Impfen der Gießschmelzenprobe vorgesehen ist, **dadurch gekennzeichnet, dass** das Impfmittel (8) mittels eines Klebstoffs (7) in der Ausnehmung (3) fixiert ist, der sich neutral gegenüber dem Impfmittel (8) und der Schmelze verhält.

2. Probentiegel nach Anspruch 1, **dadurch gekennzeichnet, dass** das Impfmittel (8) als fester, separat vorgefertigter Körper oder in schüttfähiger Form bereitgestellt ist.

3. Probentiegel nach Anspruch 1 oder 2, **dadurch gekennzeichnet, dass** der Klebstoff (7) ein Saccharid ist.

4. Probentiegel nach Anspruch 3, **dadurch gekennzeichnet, dass** der Klebstoff (7) eine Glukose, ein Fruchtzucker, ein Rohr- oder Rübenzucker oder Stärke ist.

5. Probentiegel nach einem der voranstehenden Ansprüche, **dadurch gekennzeichnet, dass** das Impfmittel (8) mittels des Klebstoffs (7) an der Bodenfläche (6) der Ausnehmung (3) fixiert ist.

6. Probentiegel nach einem der voranstehenden Ansprüche, **dadurch gekennzeichnet, dass** das Impfmittel (8) als fester Körper vorliegt und scheiben-, quader- oder würfelförmig ist.

7. Probentiegel nach einem der voranstehenden Ansprüche, **dadurch gekennzeichnet, dass** das Impfmittel (8) schüttfähig ist und als Pulver oder Granulat vorliegt.

8. Probentiegel nach Anspruch 7, **dadurch gekennzeichnet, dass** das Impfmittel in den Klebstoff (7) eingegossen ist.

9. Probentiegel nach einem der voranstehenden Ansprüche, **dadurch gekennzeichnet, dass** die Ausnehmung (3) des Probentiegelgefäßes (2) von Feuerfestwerkstoff umgeben ist.

10. Probentiegel nach Anspruch 9, **dadurch gekennzeichnet, dass** das Probentiegelgefäß (2) aus Feuerfestwerkstoff besteht.

11. Verfahren zur Thermoanalyse einer Gießschmelzenprobe, umfassend folgende Arbeitsschritte:
a) Bereitstellen eines separat vorgefertigten Impfmittels (8);
b) Bereitstellen eines separat vorgefertigten Probentiegelgefäßes (2), das eine Ausnehmung (3) zur Aufnahme der Gießschmelzenprobe aufweist;
c) Fixieren des Impfmittels (8) an einer bestimmten Position (X) innerhalb der Ausnehmung (3) des Probentiegelgefäßes (2) mittels eines Klebstoffs (7), der sich in Bezug auf das Impfmittel und die Gießprobenschmelze neutral verhält.

12. Verfahren nach Anspruch 11, **dadurch gekennzeichnet, dass** der Klebstoff (7) ein Saccharid ist.

13. Verfahren nach Anspruch 12, **dadurch gekennzeichnet, dass** das Impfmittel (8) mit dem Kleber (7) übergossen wird.

14. Verfahren nach einem der Ansprüche 11 bis 13,
**dadurch gekennzeichnet, dass** der Klebstoff (7) eine Glukose, ein Fruchtzucker, ein Rohr- oder Rübenzucker oder Stärke ist.

15. Verfahren nach einem der Ansprüche 11 - 14,
**dadurch gekennzeichnet, dass** die Gießschmelze, aus der die Gießschmelzenprobe entnommen wird, eine Eisengussschmelze ist.
